# EUROPÄISCHE PATENTSCHRIFT

(11) **EP 1 200 095 B1**
(45) Veröffentlichungstag und Bekanntmachung des Hinweises auf die Patenterteilung: **19.10.2005**
(21) Anmeldenummer: 00947960.1
(22) Anmeldetag: 10.07.2000
(51) Int. Cl.: A61K 31/55, A61K 45/06, A61K 31/70, A61P 43/00

(54) **ARZNEIMITTEL MIT PROTEKTIVER WIRKUNG GEGEN OXIDATIV-TOXISCHE UND INSBESONDERE GEGEN KARDIOTOXISCHE SUBSTANZEN**
MEDICAMENT WITH A PROTECTIVE EFFECT AGAINST OXIDATIVE-TOXIC SUBSTANCES, PARTICULARLY AGAINST CARDIOTOXIC SUBSTANCES
MEDICAMENTS A EFFET PROTECTEUR CONTRE DES SUBSTANCES TOXIQUES OXYDATIVEMENT ET EN PARTICULIER CONTRE DES SUBSTANCES CARDIOTOXIQUES

(30) Priorität: 13.07.1999 DE 19932555
(43) Veröffentlichungstag der Anmeldung: 02.05.2002
(73) Patentinhaber: Solvay Pharmaceuticals GmbH, 30173 Hannover (DE)
(72) Erfinder: ROZSA, Zsuzsanna, H-6722 Szeged (HU); PAPP, Julius, Gy., H-6722 Szeged (HU); THORMÄHLEN, Dirk, D-31039 Rheden (DE); WALDECK, Harald, D-30916 Isernhagen (DE)
(74) Vertreter: Gosmann, Martin
(86) Internationale Anmeldenummer: PCT/EP2000/006525
(87) Internationale Veröffentlichungsnummer: WO 2001/003699

(56) Entgegenhaltungen:
- WO-A-99/13871
- DE-A- 19 510 566
- DE-A- 19 638 020

## Beschreibung

Die vorliegende Erfindung betrifft die Verwendung von Benzazepin-N-essigsäurederivaten, welche in α-Stellung zu dem Stickstoffatom eine Oxogruppe enthalten und in 3-Stellung durch einen 1-(Carboxyalkyl)-cyclopentyl-carbonyl-amino-Rest substituiert sind, und deren Salzen und biolabilen Estern zur Herstellung von pharmazeutischen Zubereitungen insbesondere zur Prophylaxe und/oder Behandlung von Schädigungen des Herzens in Menschen, welche durch oxidativ-toxische, insbesondere kardiotoxische Dosen von Arzneimitteln verursacht werden. Die Erfindung betrifft hierbei allgemein auch die Verwendung der genannten Benzazepin-N-essigsäurederivate zur Herstellung von pharmazeutischen Zubereitungen zur adjuvanten Behandlung im Rahmen von Therapien, bei denen Arzneimittel mit oxidativ-toxischen, und insbesondere kardiotoxischen Nebenwirkungen eingesetzt werden. Bevorzugt betrifft die Erfindung die Prophylaxe und Behandlung von Schädigungen des Herzens, insbesondere des Myokards, welche im Rahmen einer zytostatischen Chemotherapie auftreten können.

Es ist bekannt, daß die in der Chemotherapie von malignen Tumoren verwendeten Zytostatika als unerwünschte Nebenwirkung kardiotoxische Eigenschaften aufweisen können. So werden im Rahmen der zytostatischen Therapie auch einige Antibiotika eingesetzt, die wegen ihrer allgemeinen toxischen Eigenschaften nicht zur Behandlung bakterieller Infektionen eingesetzt werden können. Hierunter fallen beispielsweise die aus Streptomyces-Arten isolierten Anthracycline, die zu den wichtigen neueren Entwicklungen auf dem Gebiet der Zytostatika zählen. Die klinische Verwendbarkeit der Anthracycline ist jedoch durch ihre mehr oder weniger stark ausgeprägte Kardiotoxizität eingeschränkt. Die Kardiotoxizität ist hierbei mit der applizierten Gesamtdosis korreliert und häufig irreversibel. Vermutlich beruht die Herzschädigung ebenso wie die zytostatischen Effekte dieser Antibiotika zumindest teilweise auf deren Membranwirkung, durch die über eine Bindung des Antibiotikums an Bestandteile der Zellmembran die Membranfluidität und -permeabilität erhöht wird. Ferner kann als weitere Ursache eine oxidative Schädigung mit in Betracht gezogen werden.

Typische, in der zytostatischen Therapie eingesetzte Antibiotika sind die Anthracycline Daunorubicin und dessen Prodrug Zorubicin, Doxorubicin (Adriamycin) und Epirubicin, sowie das synthetische Antibiotikum Mitoxantron.

Benzazepin-N-essigsäurederivate, welche in α-Stellung zu dem Stickstoffatom eine Oxogruppe enthalten und in 3-Stellung durch einen 1-(Carboxyalkyl)-cyclopentyl-carbonyl-amino-Rest substituiert sind, und deren Salzen und biolabilen Ester fallen unter den Schutzumfang von in der deutschen Patentanmeldung DE 195 10 566 beschriebenen Benzazepin-, Benzoxazepin- und Benzothiazepin-N-essigsäurederivaten, welche in α-Stellung zu dem Stickstoff eine Oxogruppe enthalten und in 3-Stellung durch einen 1-(Carboxyalkyl)-cyclopentylcarbonylamino-Rest substituiert sind, und NEP-inhibitorische Wirkungen am Herzen besitzen. Die hier im Rahmen der vorliegenden Erfindung verwendeten Benzazepin-N-essigsäure-Verbindungen können nach den in der DE 195 10 566 beschriebenen Verfahren hergestellt werden.

Der Erfindung liegt die Aufgabe zugrunde, neue pharmazeutische Zubereitungen zur Prophylaxe und/oder Behandlung von am Herzen im Zusammenhang mit der Anwendung von kardiotoxischen Dosen von Arzneimitteln auftretenden Schädigungen zu entwickeln.

Erfindungsgemäß werden nun Verbindungen der allgemeinen Formel I 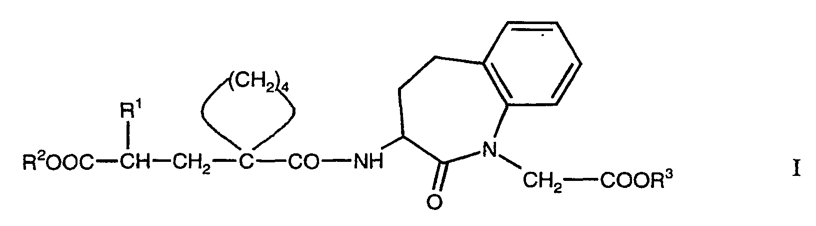 worin
- R¹: für eine Phenylniederalkylgruppe, welche gegebenenfalls im Phenylring durch niederes Alkyl, niederes Alkoxy oder Halogen substituiert sein kann, oder für eine Naphthylniederalkylgruppe steht,
- R²: Wasserstoff oder eine einen biolabilen Ester bildende Gruppe bedeutet und
- R³: Wasserstoff oder eine einen biolabilen Ester bildende Gruppe bedeutet
und physiologisch verträgliche Salze der Säuren der Formel I zur Herstellung von pharmazeutischen Zubereitungen zur Prophylaxe und/oder adjuvanten Behandlung in Therapien, bei denen kardiotoxische, eine Dosen Schädigung des Herzens, insbesondere des Myokards, induzierende von Zytostatika, vorzugsweise von zytostatisch wirksamen Antibiotika, in Menschen eingesetzt werden.

Ferner werden die Verbindungen der vorstehenden allgemeinen Formel I und von physiologisch verträglichen Salzen von Säuren der Formel I zur Herstellung von pharmazeutischen Zubereitungen zur adjuvanten Behandlung bei Menschen in Therapien verwendet, bei denen Arzneimittel mit oxidativ-zytotoxischen, insbesondere oxidativ-kardiotoxischen, Nebenwirkungen eingesetzt werden.

Sofern in den Verbindungen der Formel I die Substituenten niedere Alkyl- oder Alkoxygruppen bedeuten oder enthalten, können diese geradkettig oder verzweigt sein und insbesondere 1 bis 4, vorzugsweise 1 bis 2, Kohlenstoffatome enthalten und stellen bevorzugt Methyl oder Methoxy dar. Sofern die Substituenten Halogen enthalten, kommen insbesondere Fluor, Chlor oder Brom, vorzugsweise Fluor oder Chlor in Frage.

In dem Rest R¹ kann die Niederalkylenkette 1 bis 4, vorzugsweise 1 bis 2 Kohlenstoffatome enthalten. Insbesondere stellt R¹ eine gegebenenfalls substituierte Phenethylgruppe dar, welche gegebenenfalls ein- oder mehrfach durch Halogen, niederes Alkoxy oder niederes Alkyl substituiert sein kann, oder eine Naphthylethylgruppe.

Die Verbindungen der Formel I stellen gegebenenfalls veresterte Dicarbonsäurederivate dar. Je nach Applikationsform sind biolabile Monoester, insbesondere Verbindungen, worin R² eine einen biolabilen Ester bildende Gruppe und R³ Wasserstoff bedeuten, oder Dicarbonsäuren bevorzugt, wobei letztere insbesondere für i.v.-Applikation geeignet sind.

Als biolabile Ester bildende Gruppen R² und R³ eignen sich niedere Alkylgruppen, gegebenenfalls im Phenylring durch niederes Alkyl oder durch eine an zwei.benachbarte Kohlenstoffatome gebundene niedere Alkylenkette substituierte Phenyl- oder Phenylniederalkylgruppen, im Dioxolanring gegebenenfalls durch niederes Alkyl substituierte Dioxolanylmethylgruppen oder gegebenenfalls an der Oxymethylgruppe durch niederes Alkyl substituierte C₂-C₆-Alkanoyloxymethylgruppen. Sofern die einen biolabilen Ester bildende Gruppe R² oder R³ niederes Alkyl bedeutet, kann dieses eine bevorzugt unverzweigte Alkylgruppe mit 1 bis 4, vorzugsweise 2 Kohlenstoffatomen darstellen. Sofern die einen biolabilen Ester bildende Gruppe eine gegebenenfalls substituierte Phenylniederalkylgruppe darstellt, kann deren Alkylenkette 1 bis 3, vorzugsweise 1, Kohlenstoffatome enthalten. Sofern der Phenylring durch eine niedere Alkylenkette substituiert ist, kann diese 3 bis 4, insbesondere 3 Kohlenstoffatome enthalten. Als phenylhaltige Substituenten R² und/oder R³ eignen sich insbesondere Phenyl, Benzyl oder Indanyl. Sofern R² und/oder R³ eine gegebenenfalls substituierte Alkanoyloxymethylgruppe darstellen, kann deren Alkanoyloxygruppe 2 bis 6, vorzugsweise 3 bis 5, Kohlenstoffatome enthalten und ist vorzugsweise verzweigt und kann beispielsweise einen Pivaloyloxymethylrest (= tert.-Butylcarbonyloxymethylrest) darstellen.

Als physiologisch verträgliche Salze von Dicarbonsäuren oder Monoestern der Formel I kommen deren Alkalimetall-, Erdalkalimetall- oder Ammoniumsalze in Frage, beispielsweise Natrium- oder Kalziumsalze oder Salze mit physiologisch verträglichen, pharmakologisch neutralen organischen Aminen wie beispielsweise Diethylamin oder tert.-Butylamin.

Die Verbindungen der Formel I enthalten zwei chirale Kohlenstoffatome, nämlich das die Amidseitenkette tragende Kohlenstoffatom in 3-Stellung des Ringgerüstes und das den Rest R¹ tragende Kohlenstoffatom der Amidseitenkette. Die Verbindungen können somit in mehreren optisch aktiven stereoisomeren Formen oder als Racemat vorliegen. Gemäß der vorliegenden Erfindung können sowohl die racemischen Gemische als auch die isomerenreinen Verbindungen der Formel I verwendet werden.

Es wurde nun überraschenderweise gefunden, daß die erfindungsgemäß verwendete Gruppe der Verbindungen der Formel I und deren physiologisch verträglichen Salze der Säuren außer ihren vorbekannten NEP-inhibitorischen Eigenschaften auch die Fähigkeit besitzen, Schädigungen des Herzens durch kardiotoxische Substanzen (Wirkstoffe, Chemikalien), insbesondere ab- und umbauenden Prozessen (Remodelling) wie z.B. der myokardialen Hypertrophie und Fibrosierung, entgegenzuwirken und somit am Herzen eine Schutzwirkung gegenüber diesen kardiotoxischen Substanzen ausüben. Die Verbindungen der Formel I und deren physiologisch verträglichen Salze der Säuren besitzen somit in Bezug auf die Herzschädigung durch kardiotoxische Substanzen eine vorbeugende oder schädigungsvermindernde und somit anti-kardiotoxische Wirkung am Menschen und größeren Säugetieren. Die Verbindungen der Formel I, einschließlich deren Salze von Säuren und deren biolabile Ester, eignen sich daher zur Herstellung von pharmazeutischen Zubereitungen zur Prophylaxe und/oder Behandlung von durch kardiotoxische Dosen von Arzneimitteln induzierten Schädigungen des Herzens, insbesondere des Myokards. Die ursächlich für die Herzschädigung verantwortlichen Substanzen wie z.B. Arzneimittel können vielfältiger Art sein, z.B. die in der Chemotherapie von malignen Tumoren verwendete Zytostatika, insbesondere zytostatisch wirksame Antibiotika. Ferner wurde in diesem Zusammenhang gefunden, daß die erfindungsgemäß verwendete Gruppe von Verbindungen der Formel I ganz allgemein auch antioxidative Eigenschaften zeigen. Aus diesen Eigenschaften können vorteilhafte zytoprotektive und insbesondere kardioprotektive Wirkung resultieren, so daß sich die erfindungsgemäß verwendeten Verbindungen zur Herstellung von pharmazeutischen Zubereitungen zur adjuvanten Behandlung bei Menschen in Therapien eignen, bei denen Arzneimittel mit oxidativ-zytotoxischen und insbesondere mit oxidativ-kardiotoxischen Nebenwirkungen eingesetzt werden.

Die anti-kardiotoxische, also die gegen die durch kardiotoxische Substanzen bedingte Herzschädigung vorbeugend oder schädigungsvermindernd gerichtete, Wirkung sowie die antioxidative Wirkung der erfindungsgemäß verwendeten Verbindungen der Formel I wurde in pharmakologischen Tests in vivo an Kaninchen und Ratten mit jeweils Adriamycin-induzierter Kardiomyopathie nachgewiesen. Der Nachweis erfolgte durch Messung der Substanzwirkung an Kaninchen in Bezug auf die Hemmung bzw. Verminderung von Adriamycin-induzierten Ab- und Umbauprozessen (Remodelling) am Herzen, sowie durch Messung der Antioxidant-Aktivität der Verbindungen an Ratten.

### Beschreibung der Testmethoden

A) Die Tests wurden an Kaninchen beiderlei Geschlechts mit einem Anfangskörpergewicht von 2,1 ± 0,2 kg durchgeführt. Die Tiere wurden in 3 Gruppen eingeteilt:
1. unhandelte Tiere (= Kontrolltiere, n = 20);
2. Adriamycin-behandelte Tiere (+ Placebo statt Testsubstanz, n = 8);
3. Adriamycin- und Testsubstanz behandelte Tiere (n = 8).

Als Testsubstanz wurde stellvertretend für die erfindungsgemäß verwendbaren Substanzen der Formel I die (3S,2R')-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure eingesetzt.
Die Gruppen 2 und 3 erhielten 4 Wochen lang zweimal pro Woche 1 mg/kg Adriamycin i.v. verabreicht. Die Gruppe 3 der Kaninchen mit Adriamycin-induzierter Kardiomyopathie erhielt eine tägliche orale Dosis der Testsubstanz (30 mg/kg Körpergewicht) über 4 Wochen, beginnend mit dem ersten Tag der Adriamycin-Behandlung, mit dem Futter verabreicht. Nach Ablauf der 4 Wochen wurden die Herzen isoliert und gewogen. Anschließend wurden sie Formalin-fixiert für spätere biochemische Untersuchungen (Hydroxyprolin-Gehalt des Herzgewebes gemessen mit der HPLC Aminosäuren Analyse nach Blankenship, D.T. et al., Aunal. Biochem. 178, 227 - 232, 1989 und Schuster, R., J. Chromatogr. 431, 271 - 284, 1989). Sowohl der Anstieg des Herzgewichtes im Verhältnis zum Körpergewicht sowie des Hydroxyprolin-Gehaltes im Herzgewebe im Vergleich zu normalen Werten sind Indikatoren für am Herzen ablaufende Ab- und Umbauprozesse (Remodelling). Die Testergebnisse sind der nachfolgenden Tabelle I zusammengestellt.

**Tabelle I:**

| Verminderung der durch Adriamycin bewirkten kardialen Umbauprozesse durch die Testsubstanz in Kaninchenherzen | | | | |
|---|---|---|---|---|
| **Gemessene** **Parameter** | **Gruppe 1:** Unbehandeite Tiere, n = 20 (X ± SEM) | **Gruppe 2:** Adriamycin- + Placebo behandelte Tiere, n = 8 (X ± SEM) | **Gruppe 3:** Adriamycin + Testsubstanz behandelte Tiere, n = 8 (X ± SEM) | **% Effekt** der Testsubstanz (Gruppe 3 vs. 2) |
| Verhältnis der Herzgewichte zum Körpergewicht (g) | 2,01 ± 0,08 | 3,39 ± 0,13*** | 2,79 ± 0,08⁺ | - 17,7 |
| Hydroxyprolingehalt des Herzens (µg/ng) | 6,66 ± 0,45 | 10,68 ± 0,69*** | 9,26 ± 2,51 | - 13,3 |
| SEM = Standard Error of the Mean | | | | |

| | | | | |
|---|---|---|---|---|
| *** p < 0,001 vs. unbehandelt (Gruppe 1) | | | | |
| + p < 0,01 vs. Adriamycin + Placebo (Gruppe 2) | | | | |

Bei dieser Testmethode führte die Behandlung mit der Testsubstanz zu einem statistisch signifikanten Abfall des Herz/Körpergewicht-Verhältnisses im Vergleich zu Adriamycin-behandelten Kontrolltieren. Durch Adriamycin-Behandlung (Gruppe 2) erhöhte sich das Herz/Körpergewicht-Verhältnis (gemessen in g/kg) bezogen auf die unbehandelte Kontrollgruppe (Gruppe 1) statistisch hochsignifikant um etwa 69 %. Wurde neben Adriamycin zusätzlich die Testsubstanz verabreicht (Gruppe 3), verminderte sich der Adriamycin-induzierte Anstieg des Herz/Körpergewicht-Verhältnisses statistisch signifikant um etwa 18 % im Vergleich zu Placebo-behandelten Tieren (Gruppe 2).
Die linksventrikuläre myokardiale Hydroxyprolin-Konzentration, die ein Maß für die kardiale Fibrosierung darstellt, war bei mit Testsubstanz behandelten Tieren (Gruppe 3) geringer als bei Adriamycin-behandelten Kontrolltieren (Gruppe 2). Durch Adriamycin-Behandlung erhöhte sich der myokardiale Hydroxyprolin-Gehalt (gemessen in µg/ng) des Herzens im Vergleich zur unbehandelten Kontrollgruppe (Gruppe 1) statistisch hochsignifikant um etwa 60 %. Wurde neben Adriamycin auch die Testsubstanz verabreicht (Gruppe 3), konnte der Adriamycin-induzierte Anstieg des Hydroxyprolin-Gehaltes um etwa 13 % im Vergleich zu Placebo-behandelten Tieren (Gruppe 2) vermindert werden. Aus den Ergebnissen kann geschlossen werden, daß der Umbauprozeß der extrazellulären myokardialen Matrix durch Verabreichung der Testsubstanz deutlich reduziert wird.
B) Die Tests wurden an männlichen Wistar Ratten mit einem Anfangskörpergewicht von 229 bis 277 g durchgeführt. Die Tiere wurden in 4 Gruppen eingeteilt:
1. unbehandelte Tiere (= Kontrolltiere, n = 19);
2. Adriamycin-behandelte Tiere (+ Placebo statt Testsubstanz, n = 14);
3. Testsubstanz-behandelte Tiere (n = 11);
4. Adriamycin- und Testsubstanz-behandelte Tiere (n = 14).

Als Testsubstanz wurde stellvertretend für die erfindungsgemäß verwendbaren Substanzen der Formel I die(3S,2R')-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure eingesetzt.
Die Tiere der Gruppen 2 und 4 erhielten 15 mg/kg Adriamycin intraperitoneal über einen Zeitraum von 2 Wochen. Beginnend mit dem ersten Tag der Adriamycin-Behandlung erhielten die Tiere der Gruppe 4 über 2 Wochen täglich 30 mg/kg der Testsubstanz mit dem Futter. Die Tiere der Gruppe 3 erhielten ebenfalls täglich 30 mg/kg der Testsubstanz mit dem Futter über 2 Wochen (jedoch ohne Adriamycin).
Nach Ablauf der 2-wöchigen Behandlung wurden die Tiere mit Pentobarbital (50 mg/kg i.p.) anästhesiert und venöse Blutproben entnommen, aus denen Plasma gewonnen wurde. Die Konzentration an Lipid-Peroxiden und die Ferroxidase-Aktivität im Plasma wurde gemessen nach den Methoden von Wong, S.H.Y. et al., Clin. Chem. 33, 214-220, 1987 bzw. Johnson, D.A. et al., Clin. Chem. 13, 142-150, 1967. Weiterhin wurde nach der Methode von Catignani, G.L. und Bieri, J.G., Clin. Chem. 29, 708-712, 1983 die α-Tocopherol-Konzentration im Plasma gemessen. Die Testergebnisse sind in der nachfolgenden Tabelle II zusammengestellt. 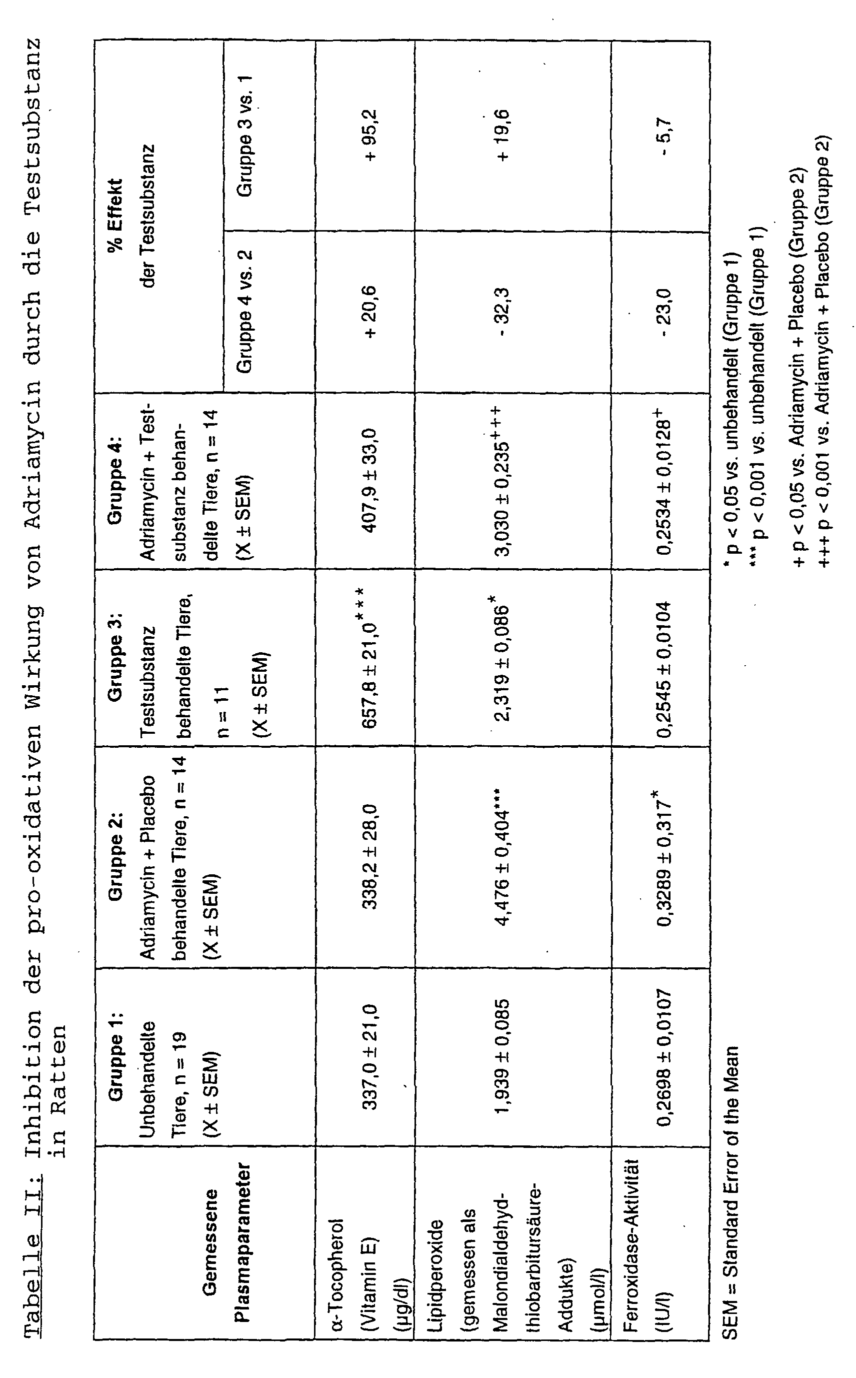
Die Testsubstanz zeigte direkt antioxidative Effekte (z.B. Erhöhung von Plasma α-Tocopherol im Vergleich zu Kontrolltieren und Adriamycin-behandelten Tieren) und inhibierte die pro-oxidative Wirkung von Adriamycin, was sich durch signifikante Reduktion der Lipidoxidation und Plasma-Ferroxidase-Aktivität im Vergleich zu Adriamycin-behandelten Vergleichsratten zeigte. Durch Verabreichung der Testsubstanz erhöhte sich der α-Tocopherol-Gehalt (Vitamin E, gemessen in µg/dl) im Plasma der Versuchstiere in Gruppe 3 im Vergleich zu den Kontrolltieren (Gruppe 1) statistisch hochsignifikant um etwa 95 %. Bei den Adriamycin- und Testsubstanz-behandelten Tieren (Gruppe 4) wurde im Vergleich zu den Tieren der Gruppe 2 (Adriamycin + Placebo) ebenfalls ein deutlicher Anstieg des α-Tocopherol-Gehaltes im Plasma um etwa 21 % festgestellt. Die Konzentration an Lipidperoxiden im Rattenplasma (gemessen als Malondialdehyd-Thiobarbitursäure-Addukte) stieg bei Adriamycin-behandelten Tieren der Gruppe 2 statistisch hochsignifikant um etwa 131 % im Vergleich zur Kontrollgruppe (Gruppe 1). Wurde neben Adriamycin zusätzlich die Testsubstanz verabreicht (Gruppe 4) konnte der Adriamycin-induzierte Anstieg der Plasmakonzentration an Lipidperoxiden statistisch hochsignifikant um etwa 32 % im Vergleich zur Gruppe 2 (Adriamycin + Placebo) vermindert werden. Die Gesamtaktivität der Ferroxidase (gemessen in IU/l) im Rattenplasma stieg in der Adriamycin-behandelten Gruppe (Gruppe 2) statistisch signifikant um etwa 22 % im Vergleich zur Kontrollgruppe (Gruppe 1). Wurde neben Adriamycin zusätzlich die Testsubstanz verabreicht (Gruppe 4) ging die Ferroxidase-Aktivität statistisch signifikant um 23 % im Vergleich zur Gruppe 2 (Adriamycin + Placebo) zurück, und entsprach damit in etwa der Ferroxidase-Aktivität, die für die Kontrollgruppe (Gruppe 1) ermittelt worden war.

Aus diesen Testergebnissen kann geschlossen werden, daß die pro-oxidative Wirkung von Adriamycin bei der durch diese Substanz bewirkten Kardiotoxizität eine Rolle spielt, und daß die Testsubstanz diese Kardiotoxizität durch ihre anti-oxidativen Eigenschaften positiv beeinflußt.

Aufgrund ihrer vorstehend beschriebenen Wirkung sind die Verbindungen der Formel I geeignet als Arzneimittel für Menschen zur Prophylaxe und/oder Behandlung von durch schädigende Einflüsse von kardiotoxischen Dosen von Arzneimitteln bedingten Schädigungszuständen am Herzen, z.B. insbesondere Ab- und Umbauprozesse (Remodelling) am Herzen wie myokardiale Hypertrophie oder Fibrosierung. Die Verbindungen der allgemeinen Formel I zeigen dabei auch eine vorteilhafte antioxidative Wirkung. Dadurch können schädigende oxidative Einflüsse von anderen Arzneimitteln, wie z.B. von Zytostatika vermindert werden. Die Verbindungen der Formel I können somit als Arzneimittel zur adjuvanten Behandlung in solchen Therapien eingesetzt werden, bei denen Arzneimittel mit oxidativ-toxischen, insbesondere kardiotoxischen Nebenwirkungen verabreicht werden. Hierbei werden Dicarbonsäuren der Formel I und deren Salze zweckmäßig in parenteral, insbesondere i.v., applizierbaren Arzneiformen und Mono- oder Diester der Formel I zweckmäßig in oral applizierbaren Arzneiformen eingesetzt. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden, unter Verwendung üblicher fester oder flüssiger Trägerstoffe wie z.B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die Erfindung betrifft auch Erzeugnisse, die ein kardiotoxische Nebenwirkungen oder ein oxidativ-zytotoxische oder oxidativ-kardiotoxische Nebenwirkungen aufweisendes Arzneimittel, insbesondere ein kardiotoxische Nebenwirkungen aufweisendes Zytostatikum, und eine Verbindung der oben beschriebenen Formel I oder ein physiologisch verträgliches Salz von Säuren der Formel I als Kombinationspräparat zur gleichzeitigen, getrennten oder abgestuften Anwendung in einer Therapie mit dem kardiotoxische Nebenwirkungen aufweisenden Arzneimittel enthalten. Insbesondere enthalten diese Erzeugnisse als Zytostatikum ein zytostatisch wirksames Antibiotikum und eine Verbindung der Formel I oder ein physiologisch verträgliches Salz von Säuren der Formel I als Kombinationspräparat zur gleichzeitigen, getrennten oder abgestuften Anwendung in der zytostatischen Chemotherapie. Solche Erzeugnisse können beispielsweise als Antibiotikum ein zytostatisch wirksames Antibiotikum aus der Gruppe der Anthracycline, Mitoxantron oder ein Prodrug derselben enthalten. Hierbei kann das Anthracyclin insbesondere Daunorubicin, Doxorubicin (Adriamycin) oder Epirubicin, vorzugsweise Doxorubicin (Adriamycin) sein.

Die nachfolgenden Beispiele sollen die Erfindung näher erläutern.

Die nachfolgenden Beispiele 1 und 2 beschreiben erfindungsgemäße pharmazeutische Zubereitungen enthaltend einen Wirkstoff der Formel I sowie die Herstellung solcher pharmazeutischer Zubereitungen. Die erfindungsgemäß verwendeten Verbindungen der Formel I können hierfür nach den in der vorgenannten deutschen Patentanmeldung DE 195 10 566 beschriebenen Methoden hergestellt werden.

### Beispiel 1:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure enthaltende Tabletten

Man stellte Tabletten in folgender Zusammensetzung pro Tablette her:

| | |
|---|---|
| (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

### Beispiel 2:

### (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure enthaltende Injektionslösung.

Man stellte eine Injektionslösung mit folgender Zusammensetzung pro 5 ml her:

| | |
|---|---|
| (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure | 10 mg |
| Na₂HPO₄·7H₂O | 43,24 mg |
| Na₂HPO₄·2H₂O | 7,72 mg |
| NaCl | 30,0 mg |
| gereinigtes Wasser | 4948,0 mg |

Die Feststoffe wurden in Wasser gelöst, die Lösung wurde sterilisiert und in Portionen von jeweils 5 ml in Ampullen abgefüllt.

### Beispiel 3:

Bevorzugte Verbindungen der Formel I für die erfindungsgemäße Verwendung zur Herstellung von Arzneimitteln zur Prophylaxe und/oder Behandlung von Schädigungen des Herzens, welche durch oxidativ-toxische und insbesondere kardiotoxische Dosen von Arzneimitteln verursacht werden, insbesondere zur adjuvanten Behandlung im Rahmen von Therapien mit derartigen Arzneimitteln, wie z.B. im Rahmen einer zytostatischen Chemotherapie, sind z.B. (einschließlich der Salze von Säuren):
3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.
3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.
(3S,2'R)-3-{1-[2'-Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.
(3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.
3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-tert.butylester.
3-[1-(2'-Carboxy-4'-phenyl-butyl)-cyclopentan-1-carbonylamino]-2,3,4,5-tetrahydro-2-oxo-1H-1-benzazepin-1-essigsäure.
3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-benzylester.
3-[1-(2'-Carboxy-4'-phenyl-butyl)-cyclopentan-1-carbonylamino]-2,3,4,5-tetrahydro-2-oxo-1H-1-benzazepin-1-essigsäure-benzylester.
3-{1-[2'-(tert.-Butylcarbonyloxymethoxycarbonyl)-4'-phenyl-butyl]-cyclopentan-1-carbonylamino}-2,3,4,5-tetrahydro-2-oxo-1H-1-benzazepin-1-essigsäure-benzylester.
3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäure.

## Patentansprüche

1. Verwendung von Verbindungen der allgemeinen Formel I 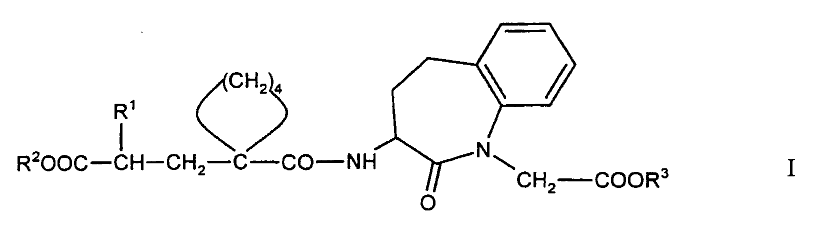 worin
R¹ für eine Phenyl-C1-4-alkylgruppe, welche gegebenenfalls im Phenylring durchC1-4-Alkyl, C1-4-Alkoxy oder Halogen substituiert sein kann, oder für eine Naphthyl-C1-4-alkylgruppe steht,
R² Wasserstoff oder eine einen biolabilen Ester bildende Gruppe bedeutet und
R³ Wasserstoff oder eine einen biolabilen Ester bildende Gruppe bedeutet,
und physiologisch verträglichen Salzen der Säuren der Formel I zur Herstellung von pharmazeutischen Zubereitungen zur Prophylaxe und/oder adjuvanten Behandlung in Therapien, bei denen kardiotoxische, eine Schädigung des Herzens, insbesondere des Myokards, induzierende Dosen von Zytostatika, vorzugsweise von zytostatisch wirksamen Antibiotika, in Menschen eingesetzt werden.

2. Verwendung von Verbindungen der allgemeinen Formel I gemäß Anspruch 1, wobei die pharmazeutischen Zubereitungen zur Prophylaxe und/oder adjuvanten Behandlung der kardiotoxischen Effekte bei der, auf der Gabe von Zytostatika basierenden Chemotherapie von malignen Tumoren geeignet sind.

3. Verwendung von Verbindungen der in Anspruch 1 angegebenen allgemeinen Formel I und von physiologisch verträglichen Salzen der Säuren der Formel I zur Herstellung von pharmazeutischen Zubereitungen zur adjuvanten Behandlung von den durch die Gabe von Zytostatika, vorzugsweise zytostatisch wirksamen Antibiotika, induzierten oxidativ-zytotoxischen, insbesondere oxidativ-kardiotoxischen, Nebenwirkungen in Therapien bei Menschen, bei denen solche Zytostatika eingesetzt werden.

4. Verwendung von Verbindungen der allgemeinen Formel I gemäß Anspruch 1 bis 3, worin R² und/oder R³ eine einen biolabilen Ester bildende Gruppe bedeuten.

5. Verwendung von Verbindungen der allgemeinen Formel I nach einem der vorstehenden Ansprüche, worin die einen biolabilen Ester bildende Gruppe eine C₁₋₄-Alkylgruppe darstellt, eine gegebenenfalls im Phenylring durch C₁₋₄-Alkyl oder durch eine an 2 benachbarte Kohlenstoffatome gebundene, C₃₋₄-Alkylenkette substituierte Phenyl- oder Phenyl-C₁₋₃-alkylgruppe, insbesondere Phenyl, Benzyl oder Indanyl, darstellt, eine im Dioxolanring gegebenfalls durch C₁₋₄-Alkyl substituierte Dioxolanylmethylgruppe, insbesondere (2,2-Dimethyl-1,3-dioxolan-4-yl)methyl, darstellt, oder eine gegebenenfalls an der Oxymethylgruppe durch C₁₋₄-Alkyl substituierte C₂-C₆-Alkanoyloxymethylgruppe darstellt.

6. Verwendung von Verbindungen der allgemeinen Formel I nach einem der vorstehenden Ansprüche **dadurch gekennzeichnet, dass** R² eine einen biolabilen Ester bildende Gruppe bedeutet und R³ Wasserstoff ist.

7. Verwendung von Verbindungen gemäß Anspruch 5, **dadurch gekennzeichnet, dass** (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure oder deren physiologisch verträgliche Salze eingesetzt werden.

8. Erzeugnisse enthaltend ein kardiotoxische oder ein oxidativ-zytotoxische oder oxidativ-kardiotoxische Nebenwirkungen aufweisendes Zytostatikum, insbesondere ein kardiotoxische Nebenwirkungen aufweisendes zytostatisch wirksames Antibiotikum, und eine Verbindung der Formel I gemäß Anspruch 1 oder ein physiologisch verträgliches Salz von Säuren der Formel I als Kombinationspräparat zur gleichzeitigen, getrennten oder abgestuften Anwendung, in einer Therapie mit dem kardiotoxische oder oxidativ-zytotoxische oder oxidativ-kardiotoxische Nebenwirkungen aufweisenden Zytostatikum.

9. Erzeugnisse nach Anspruch 8, enthalten als Zytostatikum ein zytostatisch wirksames Antibiotikum und eine Verbindung der Formel I gemäß Anspruch 1 oder ein physiologisch verträgliches Salz von Säuren der Formel I als Kombinationspräparat zur gleichzeitigen, getrennten oder abgestuften Anwendung in der, zytostatischen Chemotherapie.

10. Erzeugnisse nach Anspruch 9, **dadurch gekennzeichnet, dass** sie ein zytostatisch wirksames Antibiotikum aus der Gruppe der Anthracycline oder Mitoxantron enthalten.

11. Erzeugnisse nach Anspruch 10, **dadurch gekennzeichnet, daß** das Anthracyclin Daunorubicin, Doxorubicin (Adriamycin) oder Epirubicin, vorzugsweise Doxorubicin (Adriamycin), ist.

## Claims

1. The use of compounds of the general formula I 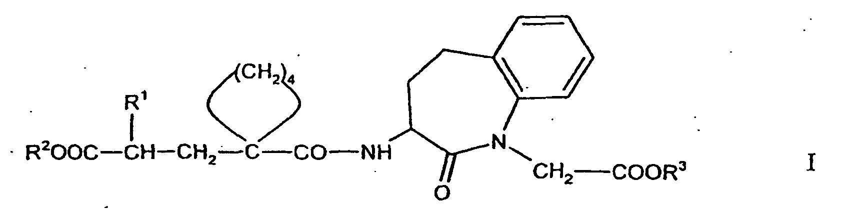 wherein
R¹ stands for a phenyl C1-4-alkyl group which may optionally be substituted in the phenyl ring by C1-4-alkyl, C1-4-alkoxy or halogen, or for a naphthyl C1-4-alkyl group,
R² is hydrogen or a group forming a biolabile ester, and
R³ is hydrogen or a group forming a biolabile ester,
and physiologically compatible salts of the acids of Formula I are used for the preparation of pharmaceutical preparations for prophylaxis and/or adjuvant treatment in therapies in which cardiotoxic doses, which induce damage to the heart, in particular to the myocardium, of cytostatic agents, preferably of cytostatic antibiotics, in humans.

2. The use of compounds of the general formula I according to Claim 1, wherein the pharmaceutical preparations are suitable for the prophylaxis and/or adjuvant treatment of the cardiotoxic effects of the chemotherapy of malignant tumours which is based on the administration of cytostatic agents.

3. The use of compounds of the general formula I set forth in Claim 1 and of physiologically compatible salts of the acids of Formula I for the preparation of pharmaceutical preparations for adjuvant treatment of the oxidative-cytotoxic, in particular oxidative-cardiotoxic, side-effects induced by the administration of cytostatic agents, preferably cytostatic antibiotics, in therapies in humans in which such cytostatic agents are used.

4. The use of compounds of the general formula I according to Claims 1 to 3, wherein R² and/or R³ is/are a group forming a biolabile ester.

5. The use of compounds of the general formula I according to one of the preceding claims, wherein the group forming a biolabile ester is a C₁₋₄-alkyl group, or a phenyl or phenyl C₁₋₃-alkyl group which is optionally substituted in the phenyl ring by C₁₋₄-alkyl or by a C₃₋₄-alkylene chain bonded to 2 adjacent carbon atoms, in particular phenyl, benzyl or indanyl, a dioxolanylmethyl group which is optionally substituted in the dioxolane ring by C₁₋₄-alkyl, in particular (2,2-dimethyl-1,3-dioxolan-4-yl)methyl, or a C₂-C₆-alkanoyloxymethyl group optionally substituted at the oxymethyl group by C₁₋₄-alkyl.

6. The use of compounds of the general formula I according to one of the preceding claims, **characterised in that** R² is a group forming a biolabile ester and R³ is hydrogen.

7. The use of compounds according to Claim 5, **characterised in that** (3S,2'R)-3-{1-[2'-(ethoxycarbonyl)-4'-phenyl-butyl]-cyclopentane-1-carbonylamino}-2,3,4,5-tetrahydro-2-oxo-1H-1-benzazepine-1-acetic acid or the physiologically compatible salts thereof is used.

8. Products containing a cytostatic agent having cardiotoxic or oxidative-cytotoxic or oxidative-cardiotoxic side-effects, in particular a cytostatic agent having cardiotoxic side-effects, and a compound of Formula I according to Claim 1 or a physiologically compatible salt of acids of Formula I as a combination preparation for simultaneous, separate or step-wise application in therapy with the cytostatic agent having cardiotoxic or oxidative-cytotoxic or oxidative-cardiotoxic side-effects.

9. Products according to Claim 8, containing as cytostatic agent a cytostatic antibiotic and a compound of Formula I according to Claim 1 or a physiologically compatible salt of acids of Formula I as a combination preparation for simultaneous, separate or step-wise application in cytostatic chemotherapy.

10. Products according to Claim 9, **characterised in that** they contain a cytostatic antibiotic from the group consisting of the anthracyclines, or mitoxantrone.

11. Products according to Claim 10, **characterised in that** the anthracycline is daunorubicin, doxorubicin (adriamycin) or epirubicin, preferably doxorubicin (adriamycin).

## Revendications

1. Utilisation de composés de formule générale 1 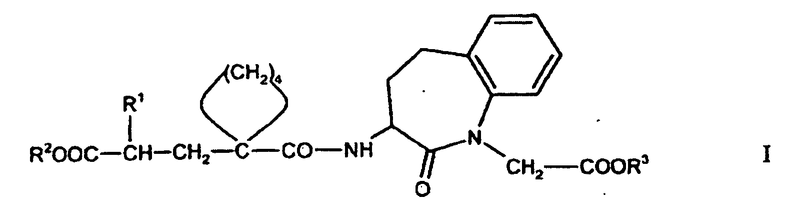 dans laquelle
R¹ est un groupe phényl-alkyle en C₁₋₄ qui peut éventuellement être substitué dans le cycle phényle par un alkyle en C₁₋₄, un alcoxy en C₁₋₄ ou un halogène, ou représente un groupe naphtyl-alkyle en C₁₋₄,
R² est un hydrogène ou représente un groupe formant un ester biolabile, et
R³ est un hydrogène ou un groupe formant un ester biolabile,
et des sels physiologiquement compatibles des acides de formule I, pour fabriquer des préparations pharmaceutiques destinées à la prophylaxie et/ou au traitement adjuvant dans des thérapies dans lesquelles on utilise chez les humains des doses de cytostatiques cardiotoxiques, induisant une lésion cardiaque, en particulier du myocarde, de préférence d'antibiotiques à effet cytostatique.

2. Utilisation de composés de formule générale I selon la revendication 1, dans laquelle les préparations pharmaceutiques destinées à la prophylaxie et/ou au traitement adjuvant des effets cardiotoxiques sont appropriées dans le cadre d'une chimiothérapie de tumeurs malignes reposant sur la prise de cytostatiques.

3. Utilisation de composés de formule générale I indiquée dans la revendication 1 et de sels physiologiquement compatibles des acides de formule I pour fabriquer des préparations pharmaceutiques destinées au traitement adjuvant des effets secondaires cytotoxiques par oxydation, en particulier cardiotoxiques par oxydation, induits par la prise de cytostatiques, de préférence d'antibiotiques à effet cytostatique, dans des thérapies chez l'homme dans lesquelles de tels cytostatiques sont utilisés.

4. Utilisation de composés de formule générale I selon la revendication 1 à 3, dans laquelle R² et/ou R³ représentent un groupe formant un ester biolabile.

5. Utilisation de composés de formule générale I selon l'une des revendications précédentes, dans laquelle le groupe formant un ester biolabile représente un groupe alkyle en C₁₋₄, un groupe phényle ou phényl-alkyle en C₁₋₃, éventuellement substitué dans le cycle phényle par un alkyle en C₁₋₄ ou par une chaîne alkylène en C₃₋₄ liée à deux atomes de carbone voisins, en particulier phényle, benzyle ou indanyle, représente un groupe dioxolanylméthyle éventuellement substitué dans le cycle dioxolane par un alkyle en C₁₋₄, en particulier (2,2-diméthyl-1,3-dioxolan-4-yl)-méthyle, ou représente un groupe (alcanoyle en C₂-C₆)-oxyméthyle éventuellement substitué dans le groupe oxyméthyle par un alkyle en C₁₋₄.

6. Utilisation de composés de formule générale I selon l'une des revendications précédentes, **caractérisée en ce que** R² représente un groupe formant un ester biolabile et R³ représente l'hydrogène.

7. Utilisation de composés selon la revendication 5, **caractérisé en ce que** l'on utilise de l'acide (3S,2'R)-3-{1-[2'-(éthoxycarbonyl)-4'-phényl-butyl]-cyclopentane-1-carbonylamino}-2,3,4,5-tétrahydro-2-oxo-1H-1-benzazépin-1-acétique ou ses sels physiologiquement compatibles.

8. Produits contenant un cytostatique présentant des effets secondaires cardiotoxiques, ou cytotoxiques par oxydation ou cardiotoxiques par oxydation, en particulier un antibiotique à effet cytostatique présentant des effets secondaires cardiotoxiques, et un composé de formule I selon la revendication 1 ou un sel physiologiquement compatible d'acides de formule I sous forme d'une préparation combinée, destinée à une administration simultanée, séparée ou échelonnée, dans une thérapie utilisant le cytostatique présentant des effets secondaires cardiotoxiques ou cytotoxiques par oxydation ou cardiotoxiques par oxydation.

9. Produits selon la revendication 8, contenant en tant que cytostatique un antibiotique à effet cytostatique et un composé de formule I selon la revendication 1 ou un sel physiologiquement compatible d'acides de formule I sous forme d'une préparation combinée destinée à une administration simultanée, séparée ou échelonnée, dans la chimiothérapie cytostatique.

10. Produits selon la revendication 9, **caractérisés en ce qu'**ils contiennent un antibiotique à effet cytostatique issu du groupe des anthracyclines ou de la mitoxantrone.

11. Produits selon la revendication 10, **caractérisés en ce que** l'anthracycline est la daunorubicine, la doxorubicine (adriamycine) ou l'épirubicine, de préférence la doxorubicine (adriamycine).
